# FASCICULE DE BREVET EUROPEEN

(11) **EP 0 534 865 B1**
(45) Date de publication et mention de la délivrance du brevet: **18.09.1996**
(21) Numéro de dépôt: 92420249.2
(22) Date de dépôt: 22.07.1992
(51) Int. Cl.: C12P 13/08, A23K 1/14, C05F 7/00

(54) **Procédé de séparation de la lysine sous la forme d'une solution aqueuse et utilisation de cette solution pour l'alimentation animale**
Verfahren zum Abtrennen von Lysine in Form von einer wässerigen Lösung und Verwendung dieser Lösung in der Tierernährung
Process for separating lysine in the form of an aqueous solution and use of said solution in animal food

(30) Priorité: 26.07.1991 FR 9109778
(43) Date de publication de la demande: 31.03.1993
(73) Titulaire: EUROLYSINE, F-75009 Paris (FR)
(72) Inventeur: Lucq, Pierre, F-80480 Salouel (FR); Domont, Christian, F-80920 Contay (FR)
(74) Mandataire: Tilloy, Anne-Marie

(56) Documents cités:
- FR-A- 2 286 121
- CHEMICAL ABSTRACTS, vol. 85, no. 17, 25 Octobre 1976, Columbus, Ohio, US; abstract no. 121810c, 'Isolation of L-lysine from fermentation medium' page 482 ;colonne 2 ;
- CHEMICAL ABSTRACTS, vol. 65, no. 12, 5 Décembre 1966, Columbus, Ohio, US; abstract no. 19275e, 'Production of L-lysine' colonne 1 ;
- CHEMICAL ABSTRACTS, vol. 90, no. 19, 7 Mai 1979, Columbus, Ohio, US; abstract no. 150317f, 'Technical quality of L-lysine' page 463 ;colonne 2 ;
- PATENT ABSTRACTS OF JAPAN vol. 11, no. 259 (C-441)(2706) 21 Août 1987

## Description

La présente invention concerne un procédé de séparation de la lysine sous la forme d'une solution aqueuse à partir d'un bouillon de fermentation. Egalement, l'invention concerne les solutions de lysine base susceptibles d'être ainsi obtenues et leur utilisation pour la préparation de compositions destinées à l'alimentation animale.

Dans les procédés de préparation de lysine par fermentation, on connaît essentiellement deux familles de procédés de récupération de la lysine ayant fait l'objet d'un développement industriel.

Une première famille de procédés propose la récupération de lysine sous la forme de monochlorhydrate de lysine déhydraté à l'état cristallisé. Il est, dans ce cas, de pratique courante de récupérer la lysine à partir du bouillon de fermentation en utilisant une résine échangeuse de cations.

Pour ce faire, on procède selon les étapes principales suivantes :
1. Ajustement du pH du bouillon de fermentation, dont on a éventuellement éliminé les bactéries, dans un intervalle de valeurs optimales pour l'adsorption de la lysine sur la résine par addition d'un acide, généralement l'acide chlorhydrique ou l'acide sulfurique.
2. Passage du bouillon de fermentation acidifié sur une résine échangeuse de cations, qui adsorbe la lysine ainsi qu'une partie des cations présents telles que : ammonium, potassium, calcium et des amino-acides dérivés des substances qui se trouvent dans le bouillon de fermentation ou qui sont formés pendant la fermentation. Ces ions diminuent la capacité d'adsorption de lysine des résines car ils sont adsorbés sur celles-ci en même temps que la lysine. C'est pourquoi, pour améliorer la récupération de la lysine, on a recours à des technologies complexes et coûteuses utilisant plusieurs colonnes remplies d'une résine échangeuse de cations et disposées en série. Egalement pour favoriser cette adsorption, on ajuste le pH du bouillon de fermentation à des pH fortement acides, généralement inférieurs à 4 (se reporter par exemple au brevet français n° 2567413). La fermentation étant généralement conduite à des valeurs proches de la neutralité, une quantité importante d'acide est donc nécessaire pour porter son pH à ces valeurs.
3. Elution de la lysine adsorbée sur la résine par une solution alcaline, généralement de l'ammoniaque, et régénération de la résine.
4. Récupération de la lysine de l'éluat sous la forme de cristaux de monochlorhydrate de lysine dihydraté par concentration de la solution éluée dont le pH a été préalablement ajustée à une valeur inférieure à 6, suivie par la cristallisation, et enfin la séparation et le séchage des cristaux obtenus.

Un inconvénient majeur de cette méthode réside dans le fait qu'elle est de mise en oeuvre coûteuse. En effet :
- elle est relativement complexe et nécessite de nombreuses installations, colonnes de résines par exemple,
- l'élution de la lysine nécessite des quantités importantes d'eau.
- des quantités importantes d'eau sont également nécessaires pour régénérer la résine,
- les eaux résiduaires ainsi produites, essentiellement lors de l'élution de la lysine et de la régénération de la résine qui sont chargées en matières minérales et/ou en diverses matières organiques, doivent subir un traitement en station d'épuration avant d'être réutilisées ou rejetée dans le milieu naturel.

En conséquence, l'extraction de la lysine selon cette première méthode occasionne une dépense d'énergie importante.

En outre, ce procédé est également coûteux en raison de la consommation importante de réactifs qu'il implique.

Un deuxième famille de procédés propose la récupération de lysine sous la forme d'une composition déshydratée pouvant comprendre également la biomasse et/ou les différents constituants présents dans le milieu de fermentation.

Ainsi, il est connu du brevet SU-183581 un procédé de préparation de L-lysine pure selon lequel:
a) la fermentation est réalisée avec comme sources de carbone des acides gras de faible poids moléculaire ;
b) le bouillon de fermentation est bouilli puis débarrassé des micro-organismes ;
c) le filtrat est acidifié avec Hcl à pH 5 puis traité avec 4-5 % de cabone actif à 50-55°C pendant trente minutes avec une agitation constante : cette étape a pour but de provoquer la décoloration du filtrat ;
d) après filtration pour éliminer le carbone actif, on ajoute de l'hydroxyde de calcium, ceci afin d'éliminer le sulfate d'ammonium sous la forme de sulfate de calcium et d'hydroxyde d'ammonium, et le saccharose restant sous la forme de saccharose e calcium ;
e) la solution est filtrée, concentrée par évaporation : ceci a pour effet de décomposer l'hydroxyde d'ammonium en eau et ammoniac, ce dernier étant alors distillé ;
f) du fait de l'évaporation, on obtient des solutions très concentrées en L-lysine, qui se cristallise lors du refroidissement ;
g) les cristaux sont dissous dans une faible quantité d'eau puis précipités par ajout d'éthanol ;
h) après filtration et séchage, on recueille des cristaux de L-lysine pure.

Un tel procédé est incontestablement complexe.

En outre, ces compositions déshydratées conduisent à des solides pâteux, collants et très hygroscopiques, qui sont de ce fait difficilement manipulables. Enfin, la stabilité au stockage de ces compositions déshydratées est médiocre.

Pour résoudre ces problèmes, il a été proposé l'emploi d'additifs dans les moûts de fermentation productrice de lysine qui permettrait de limiter leur caractère hygroscopique. Cette solution n'est pas totalement satisfaisante car elle nécessite des quantités élevées d'additif, et en conséquence, conduit à la production de compositions déshydratées à teneur en lysine relativement faibles.

Il a également été proposé d'effectuer la fermentation productrice de lysine dans des conditions bien définies. En particulier dans le brevet français n° 2541867, on préconise l'utilisation de source de carbone à teneur élevée en matières fermentescibles par rapport à la matière sèche totale, c'est-à-dire une source de carbone contenant une matière carbonée oxygénée assimilable à un taux de pureté suffisamment élevé, telle que le glucose, le saccharose, les hydrolysats d'amidon. Un tel procédé est relativement coûteux à mettre en oeuvre, car il exclut l'emploi majoritairement de source de carbone bon marché, tels que les mélasses de betterave ou de canne à sucre. En outre, il nécessite une dépense d'énergie importante lors de la déshydratation.

Par ailleurs, la déshydratation doit être conduite dans des conditions particulières pour que le produit final ne soit pas dégradé. Ce procédé est donc relativement complexe.

Un but de la présente invention est de proposer un procédé de séparation de la lysine à partir d'un bouillon de fermentation, qui soit à la fois simple, économique et qui conduise à l'obtention directe de solutions aqueuses et concentrées de lysine.

Un autre but de l'invention est l'utilisation de telles solutions aqueuses et concentrées de lysine comme additif aux aliments pour animaux.

Conformément à l'invention pour atteindre ces buts, on prévoit un procédé de séparation de la lysine à partir d'un bouillon de fermentation la contenant sous la forme d'une solution aqueuse, caractérisé en ce qu'il consiste à :
a) ajuster le pH du bouillon de fermentation à une valeur comprise entre 9 et 11;
b) cristalliser les sels minéraux présents dans le bouillon de fermentation par concentration et éventuellement refroidissement de ce bouillon ;
c) séparer ces sels minéraux du bouillon de fermentation ;
d) recueillir la solution aqueuse contenant la lysine que constituent les eaux-mères de cristallisation ;
et en ce qu'il comporte également une étape visant à éliminer du bouillon de fermentation les micro-organismes ayant assuré la production de lysine, cette étape pouvant être mise en oeuvre soit avant ou après l'étape a) soit après l'étape c).

Le procédé présente l'avantage majeur de réaliser simultanément et simplement l'élimination de certains constituants du bouillon de fermentation, à savoir les microorganismes et une grande proportion des matières minérales ainsi que la concentration en lysine du milieu traité. Ceci perrnet d'obtenir des solutions stables à forte concentration en lysine (comprise entre 25 et 50 % en poids).

Egalement, on élimine du bouillon de fermentation la biomasse, ce qui permet d'aboutir à une solution aqueuse, concentrée en lysine, présentant une stabilité au stockage tout à fait satisfaisante.

Grâce au procédé selon l'invention, les solutions aqueuses et concentrées en lysine qui en sont issues sont particulièrement économiques. En effet :
- comme additif, seule une base telle que NaOH, NH₄OH ou KOH est nécessaire pour sa mise en oeuvre.
- la consommation d'eau est particulièrement réduite, voire nulle.
- aucune installation spécifique n'est nécessaire pour la mise en oeuvre de l'invention. En d'autres termes, les installations habituellement utilisées en fermentation, qui comprennent un fermenteur équipé de moyens de séparation de la biomasse et des matières minérales, ne nécessitent aucune adaptation complexe pour la mise en oeuvre du proposé selon l'invention.
- enfin, du fait de leur teneur élevée en lysine et de leur stabilité, l'exploitation et la conservation industrielles des solutions selon l'invention est à la fois simple et économique.

Un autre avantage important du procédé selon l'invention réside dans le fait qu'il ne comprend aucune opération susceptible de dégrader la lysine, telle que la déshydratation qui est largement employée dans les procédés connus.

Les compositions alimentaires contenant les solutions selon l'invention présentent une efficacité, pour la croissance des animaux, équivalente à celle des compositions comprenant du chlorhydrate de lysine purifié.
Outre sa stabilité au stockage, la solution aqueuse et concentrée de lysine issue du procédé selon l'invention possède les propriétés suivantes :
. teneur élevée en lysine base qui, à titre d'exemple, exprimée en lysine base (lysine sous la forme libre) est au moins égale à 25 % en poids lorsque la source de carbohydrate utilisée pour réaliser la fermentation est de la mélasse de betterave ou de canne à sucre,
. un rapport en poids de lysine base sur matières sèches totales élevé, généralement au moins égal à 35 % en poids,
. les solutions aqueuses et concentrées de lysine base selon l'invention sont des liquides dont le mélange avec des aliments pour animaux ne présente aucune difficulté. De plus, l'ajout des solutions selon l'invention dans des aliments composés pour animaux peut toujours être fait de telle manière que les teneurs en lysine correspondent au besoin respectif des animaux.

L'opération de séparation de la lysine doit être conduite à un pH compris entre 9 et 11, sinon on favorise la précipitation de la lysine.

Conformément à un mode de réalisation préféré, la séparation de la lysine est réalisée à partir d'un bouillon de fermentation productrice de lysine dont le pH a été ajusté à une valeur comprise entre 9,5 et 10. A ces valeurs en effet, les concentrations les plus élevées en lysine ont été atteintes.

D'une manière générale, les bouillons de fermentation connus pour produire de la lysine conviennent à l'invention. Avantageusement, on choisit les bouillons produits par la fermentation d'une source d'azote et d'une source de carbone à l'aide d'un microorganisme ayant une forte productivité en lysine, appartenant, par exemple, au genre Brevibacterium ou Corynebacterium. De préférence, comme source de carbone, on choisit les mélasses de betterave ou de canne à sucre ou les sources de carbone à teneur élevée en matières fermentescibles telles que le glucose, le saccharose, les hydrolysats d'amidon ou un mélange de deux ou plusieurs des différentes sources de carbone précitées.

Dès que la concentration en source de carbone est suffisamment faible, on arrête la fermentation et la séparation de la lysine en solution aqueuse peut être entreprise : la fermentation étant généralement réalisée à des valeurs de pH proches de la neutralité, la lysine se trouve alors essentiellement à l'état de cation monovalent, sous forme d'un sel et généralement sous forme de sulfate ou de chlorhydrate.

Conformément au mode de réalisation préféré de l'invention, dans une première étape, on élimine, par exemple par ultrafiltration, microfiltration, séparation centrifuge ou filtration, les matières en suspension et en particulier les microorganismes.

On règle ensuite le pH de la solution clarifiée à une valeur comprise entre 9 et 11, et de préférence entre 9,5 et 10, en y ajoutant une base comme NaOH, KOH et NH₄OH. Avantageusement, à titre de base, on utilise KOH ou NH₄OH ou un mélange de ces deux bases, afin de conduire à des sels minéraux pouvant être employés dans le domaine des engrais.

Ensuite, on peut procéder, dans une seconde étape, à la cristallisation des sels minéraux par concentration et éventuellement refroidissement de la solution clarifiée.

D'une manière générale, la concentration est réalisée selon des techniques bien connues pour ce type d'opération. Les conditions opératoires de cette concentration sont choisies de sorte que les composés contenus dans la solution aqueuse finale ne soient pas dégradés.

Par exemple, la concentration peut être réalisée à l'aide d'appareils d'évaporation sous vide.

Au cours de cette étape, on élimine avantageusement tous les sels dont la solubilité est inférieure à celle de la lysine. Parmi les sels qui peuvent être ainsi éliminés grâce à l'invention, on peut citer SO₄²⁻, Cl⁻, Na⁺, K⁺, NH₄⁺. Les cristaux des sels minéraux qui se forment lors de l'opération de concentration sont éliminés, par exemple, par centrifugation ou filtration. Parallèlement à l'élimination des sels minéraux, on recueille la solution mère de cristallisation contenant la lysine.

### Exemple 1 : Préparation d'une solution de lysine à partir d'un bouillon de fermentation réalisé avec, comme source de carbone, un hydrolysat d'amidon (sirop à haute teneur en glucose) qui comprend 10 % en poids de mélasse de betterave et 90 % en poids de glucose.

Le proposé de fermentation utilisant un micro-organisme producteur de Lysine et utilisant comme source de carbone un hydrolysat d'amidon (solution de glucose) conduit à l'obtention d'un moût de fermentation ayant les caractéristiques suivantes :

| | |
|---|---|
| lysine base/matière sèche totale : | 50 % |
| Sulfates : | 3 % |
| pH : | 7 |

Le moût de fermentation a été ensuite ajusté à pH 10 par addition d'une solution de potasse.

Le moût ajusté a été ensuite traité par centrifugation pour éliminer la biomasse.

Le moût ainsi traité a été ensuite concentré par évaporation sous vide et on a obtenu une suspension de cristaux de sulfate de potassium à 60° C.

La suspension de cristaux a été refroidie à 10° C dans un cristallisoir pour assurer un bon épuisement en sels minéraux.
Les sels minéraux ont été éliminés par centrifugation et une solution de Lysine base ayant les caractéristiques suivantes a été obtenue :
- liquide brun clair
- densité ; 1,30
- pH: 10
- teneur en Lysine base : 45 %
- composition centésimale :
   - matière sèche : 70 %
   - protéines : 6 %
   - matières minérales : 5,8 %
      dont ions sulfates 1,,5 %

La stabilité de cette solution a été vérifiée et la perte en titre Lysine est inférieure à 1 % après 6 mois de stockage dans des conditions de températures ambiantes (variant entre -5° C et 20° C).

Aucune altération de la qualité bactériologique ni d'apparition de précipité n'a été constatée.

Cette solution est donc caractérisée par une bonne stabilité dans le temps à tous points de vue.

### Exemple 2 : Préparation d'une solution de lysine à partir d'un bouillon de fermentation réalisé avec, comme source de carbone, 100 % en poids de mélasse de betterave.

Le même type de procédé de fermentation est mis en oeuvre mais en utilisant la mélasse de betterave comme source de carbone par le micro-organisme producteur de Lysine.

Le moût de fermentation obtenu possède la composition suivante :
- Lysine base/matière sèche totale : 25 %
- Sulfates : 3,8 %
- Potassium : 1,3 %
- pH : 7

Le moût de fermentation a été ensuite ajusté à pH 9,50 par addition d'ammoniac.

Le moût ajusté a été ensuite traité sur une installation de microfiltration tangentielle afin d'éliminer les bactéries et les matières en suspension.

Le moût ainsi obtenu a été concentré par évaporation sous vide puis refroidi à 10° C afin d'obtenir la cristallisation d'un mélange de sulfate de potassium et d'ammonium.

La séparation des cristaux de sels minéraux a conduit à l'obtention d'une solution de Lysine base ayant les caractéristiques suivantes :
- liquide brun foncé
- densité : 1,35
- pH : 9,5
- teneur en Lysine base : 25 %
- composition centésimale :
   - matière sèche : 70 %
   - protéines : 16 %
   - matières minérales : 7 %
      dont ions sulfates : 2,4 %

La stabilité de la solution a été vérifiée et la perte en titre Lysine est inférieure à 1 % après 6 mois de stockage dans des conditions de températures ambiantes (variant entre -5° C et 20° C).

Aucune altération de la qualité bactériologique ni d'apparition de précipité n'a été constatée.

Cette solution est donc caractérisée par une bonne stabilité dans le temps à tous points de vue.

## Revendications

1. Procédé de séparation de la lysine à partir d'un bouillon de fermentation la contenant, sous la forme d'une solution aqueuse, caractérisé en ce qu'il consiste à :
a) ajuster le pH du bouillon de fermentation à une valeur comprise entre 9 et 11;
b) cristalliser les sels minéraux présents dans le bouillon de fermentation par concentration et éventuellement refroidissement de ce bouillon ;
c) séparer ces sels minéraux du bouillon de fermentation ;
d) recueillir la solution aqueuse contenant la lysine que constituent les eaux-mères de cristallisation ;
et en ce qu'il comporte également une étape visant à éliminer du bouillon de fermentation les micro-organismes ayant assuré la production de lysine, cette étape pouvant être mise en oeuvre soit avant ou après l'étape a) soit après l'étape c).

2. Procédé selon la revendication 1, caractérisé en ce que le pH du bouillon de fermentation est ajusté à une valeur comprise entre 9,5 et 10.

3. Proposé selon la revendication 1 ou 2, caractérisé en ce que le pH est ajusté à l'aide de KOH ou NH₄OH.

4. Solution de lysine susceptible d'être préparée selon le procédé décrit dans l'une quelconque des revendications 1 à 3.

5. Solution de lysine selon la revendication 4, caractérisée en ce qu'elle possède les propriétés suivantes :
- une teneur en lysine base au moins égale à 25 % en poids,
- un rapport en poids de lysine base sur matières sèches totales au moins égal à 35 % en poids,
- un pH compris entre 9 et 11

6. Solution de lysine selon la revendication 5, caractérisée en ce ce que son pH est compris entre 9,5 et 10.

7. Utilisation d'une solution de lysine selon l'une quelconque des revendications 4 à 6 pour la préparation d'une composition destinée à l'alimentation animale.

## Patentansprüche

1. Verfahren zum Abtrennen des Lysins aus einer es enthaltenden Fermentationsbouillon bzw. Gärbrühe in Form einer wäßrigen Lösung,
**dadurch gekennzeichnet,** daß es darin besteht:
a) Einstellen des pH-Werts der Gärbrühe auf einen zwischen 9 und 11 enthaltenen Wert;
b) Kristallisieren der in der Gärbrühe enthaltenen mineralischen Salze durch Konzentration und eventuell Abkühlung dieser Brühe;
c) Trennen dieser mineralischen Salze von der Gärbrühe;
d) Sammeln der wäßrigen Lösung, die das Lysin enthält, das die Kristallisations-Mutterlaugen bilden;
**und dadurch,** daß es ebenfalls einen Schritt umfaßt, der darauf abzielt, aus der Gärbrühe die Mikroorganismen zu beseitigen, die die Lysinerzeugung bewirkt haben, wobei dieser Schritt entweder vor oder nach dem Schritt a) oder nach dem Schritt c) ausgeführt werden kann.

2. Verfahren nach Anspruch 1,
dadurch gekennzeichnet, daß der pH der Gärbrühe auf einen zwischen 9,5 und 10 enthaltenen Wert eingestellt wird.

3. Verfahren nach Anspruch 1 oder 2,
dadurch gekennzeichnet, daß der pH-Wert mittels KOH oder NH₄OH eingestellt wird.

4. Lysinlösung, hergestellt nach einem in irgendeinem der Ansprüche 1 bis 3 beschriebenen Verfahren.

5. Lysinlösung nach Anspruch 4,
dadurch gekennzeichnet, daß sie die folgenden Eigenschaften aufweist:
- einen Basislysingehalt von wenigstens gleich 25 Gew.-%,
- ein Gewichtsverhältnis von Basislysin zu Gesamttrockenmassen von wenigstens gleich 35 Gew.-%,
- einen zwischen 9 und 11 enthaltenen pH-Wert.

6. Lysinlösung nach Anspruch 5,
dadurch gekennzeichnet, daß ihr pH-Wert enthalten ist zwischen 9,5 und 10.

7. Verwendung einer Lysinlösung nach einem der Ansprüche 4 bis 6 für die Herstellung einer zur Viehfütterung bestimmten Zusammensetzung.

## Claims

1. Process for separating lysine, from a fermentation medium containing it, in the form of an aqueous solution, characterised in that it comprises:
a) adjusting the pH of the fermentation medium to between 9 and 11;
b) crystallising the mineral salts present in the fermentation medium by concentration and if necessary cooling said medium;
c) separating the mineral salts from the fermentation medium;
d) collecting the aqueous solution containing the lysine which the crystallisation mother liquors comprise;
and in that it also comprises a step to eliminate micro-organisms from the fermentation medium having assured the production of lysine, this step being able to be carried out either before or after step a) or after step c).

2. Process according to claim 1, characterised in that the pH of the fermentation medium is adjusted to between 9.5 and 10.

3. Process according to claim 1 or 2, characterised in that the pH is adjusted using KOH or NH₄OH.

4. Lysine solution prepared according to the process described in any one of claims 1 to 3.

5. Lysine solution according to claim 4, characterised in that it has the following properties:
- a lysine base content of at least 25 % by weight,
- a weight ratio of lysine base to total dry matter of at least 35 % by weight,
- a pH of between 9 and 11.

6. Lysine solution according to claim 5, characterised in that its pH is between 9.5 and 10.

7. Use of a lysine solution according to any one of claims 4 to 6 for the preparation of a composition intended for animal food.
